# EUROPEAN PATENT APPLICATION

(11) **EP 2 333 546 A1**
(43) Date of publication of application: **15.06.2011**
(21) Application number: 09809872.6
(22) Date of filing: 24.08.2009
(51) Int. Cl.: G01N 33/53, C12Q 1/68, G01N 33/15, G01N 33/50

(54) **METHOD FOR SCREENING FOR ANTIANGIOGENIC AGENT, AND METHOD FOR SCREENING FOR ANTIANGIOGENIC SIGNAL GENE**

(30) Priority: 28.08.2008 JP 2008220518
(71) Applicant: National University Corporation Tokyo Medical and Dental University, Bunkyo-ku Tokyo 113-8510 (JP)
(72) Inventor: SHICHIRI, Masayoshi, Tokyo 1740065 (JP)
(74) Representative: Ström & Gulliksson AB
(86) International application number: PCT/JP2009/064733
(87) International publication number: WO 2010/024221

(57) **Abstract**

Disclosed are a method for screening for an antiangiogenic agent and a method for screening for an antiangiogenic gene, both of which are achieved by detecting an antiangiogenic signal within a short time by a simple means. The method for screening for an antiangiogenic agent comprises: a candidate compound administration step of administering a candidate compound for the antiangiogenic agent to a vascular endothelial cell or a cultured cell derived from a vascular endothelial cell; a cell-maintaining step of maintaining the vascular endothelial cell or the cultured cell derived from the vascular endothelial cell to which the candidate compound has been administered; and a signal detection step of detecting the phosphorylation of a protein phosphorylated by the administration of endostatin.

## Description

### TECHNICAL FIELD

The present invention relates to a method for screening for an antiangiogenic agent, and a method for screening for an antiangiogenic signal gene.

### BACKGROUND ART

Angiogenesis, a phenomenon in which a new blood vessel is formed from a pre-existing blood vessel, is known to be intimately involved in the onset and progression of various diseases including malignant (solid) tumor, diabetic retinopathy, age-related macular degeneration, and inflammatory diseases such as rheumatoid arthritis. For example, in a solid tumor, angiogenesis provides a necessary route through which the tumor obtains the nutrients and oxygen and removes waste in order to grow. Angiogenesis also plays an important step in metastasis, a clinically important problem in the treatment of cancer, by providing a route for cancer cells to metastasize. In regard to diabetic retinopathy and age-related macular degeneration, angiogenesis itself represents the pathological state, and if left untreated it results in blindness. Inhibition of angiogenesis therefore could lead to the prevention and treatment of both diseases, and drugs for preventing and treating these diseases are being developed.

As discussed above, angiogenesis can be observed in numerous disease states, and is involved in promoting their pathological progression. Inhibiting angiogenesis, for this reason, has become the center of attention in terms of preventing and treating these disorders, and the search for substances that inhibit angiogenesis is being pursued in earnest. As a result, a number of angiogenesis inhibitors have been developed, and some of the substances are currently being tested for effectiveness in clinical trials.

Angiogenesis inhibitors, such as endostatin and angiostatin for example, have been considered to be one of the most promising drugs for tumor dormancy therapy. Because these drugs can significantly shrink solid tumors in experimental animals (non-patent publication 1) without exhibiting drug resistance after repeated doses as is often the case with traditional anti-cancer drugs (non-patent publication 2), they have been regarded as potentially becoming the ideal anti-cancer drugs with very few side effects. However, even if these compounds are approved for drug use, it is still very challenging to produce them in a quantity known to have antitumor effect. The manufacturing costs also can be prohibitively high. In fact, some pharmaceutical companies have terminated their effort of developing angiostatin, a very large molecule having a molecular weight of 50,000.

With a lower molecular weight than angiostatin, endostatin (molecular weight of approximately 20,000) has become the target of drug development, and clinical trials for its use in patients with terminal stage malignant tumors have been carried out in the US. However, its intra-cellular signal transduction mechanism remains elusive.

Endostatin inhibits the growth of endothelial cells in a low serum culturing condition, and promotes apoptosis (non-patent publication 3), however, these effects are minor ones at best. The growth potential of cancer cells are enhanced not only by genetic mutations but also by the changes in the regulation of gene expression. Cancer cells promote their own growth via both autocrine and paracrine mechanisms by producing numerous growth factors and angiogenesis promoting factors. In addition, vascularization supplies ample flow of blood to the tumor. Considering these, it has been difficult to explain why endostatin has the ability to shrink tumors at the primary as well as the metastasized sites when it only exhibits a very minor growth inhibitory effect on endothelial cells. The fact that endostatin can exert such an inhibitory effect on tumor angiogenesis even in this kind of growth promoting environment as reported, suggests that it must be able to elicit strong cellular signaling that specifically acts on endothelial cells.

Patent publication 1 for example discloses various signals elicited by endostatin that exert inhibitory effects on the expression of key genes. Due to this repressive signaling of gene expression, administering endostatin to experimental animals at a dose known to induce tumor-shrinking effect results in a marked reduction in the expression levels of early response genes expressed in cultured endothelial cells that respond to stimulation by serum, growth factor, or angiogenesis promoting factor, as well as genes involved in apoptosis, cell cycle and chemotaxis.
[Patent Document 1] Japanese Unexamined Patent Application, First Publication No. 2004-075665
[Non-Patent Publication 1] Cell, 88, pp.277-285, 1997.
[Non-Patent Publication 2] Nature, 390, pp.404-407, 1997.
[Non-Patent Publication 3] J.Biol.Chem., 274, pp.11721-11726, 1999.

### DISCLOSURE OF THE INVENTION

### Problems to be Solved by the Invention

However, the molecular mechanism for the signaling that negatively regulates the gene expression elicited by endostatin administration to cultured endothelial cells has not been elucidated to date. Patent publication 1 discloses a method for screening for an antiangiogenic agent by detecting inhibition of gene expression of various angiogenesis related genes induced by endostatin administration, however, this type of experiment requires accurate quantification of the decrease in the gene expression level, a time consuming process which is not amenable to a large scale screen through which many compounds need to be efficiently tested. Similarly, in order to identify the target genes of endostatin, whose expression levels are negatively regulated, a screening method that can rapidly and easily identify the change in such signals is highly desired.

The present invention seeks to solve the above problem, and aims at elucidating the signal transduction pathway functioning upstream of the negative regulation of angiogenesis related gene expression, and utilizing this knowledge to provide a method for rapidly identifying antiangiogenic agents that have similar actions as the agents identified in the traditional screening method, and further to provide a screening method for identifying the genes involved in inhibiting the angiogenesis.

### Means for Solving the Problems

The inventors of the present invention found that the phosphorylation of specific proteins are induced following the administration of endostatin to vascular endothelial cells, that the expression of various angiogenesis related genes are negatively regulated through these phosphorylations, and that by detecting the phosphorylation of the specific proteins, the antiangiogenesis signal can be rapidly and easily detected. The present invention came to completion based on these findings. Specifically the present invention provides the following.

In a first aspect of the present invention provided is a method for screening for an antiangiogenic agent, including: a candidate compound administration step of administering a candidate compound for the antiangiogenic agent to a vascular endothelial cell or a cultured cell derived from the vascular endothelial cell; a cell-maintaining step of maintaining the vascular endothelial cell or the cultured cell derived from the vascular endothelial cell to which the candidate compound has been administered; and a signal detection step of detecting phosphorylation of a protein phosphorylated by the administration of endostatin.

According to the invention described in the first aspect, screening for an antiangiogenic agent is carried out by detecting phosphorylation of a protein whose phosphorylation is induced by the administration of endostatin. Protein phosphorylation occurs within a short period of time after administering the candidate compound for the antiangiogenic agent to the vascular endothelial cell or the cultured cell derived form vascular endothelial cell. Therefore, screening can be carried out efficiently even with a large number of candidate molecules.

In a second aspect of the method for screening for the antiangiogenic agent described in the first aspect, the protein phosphorylated by the administration of endostatin is a double stranded RNA-dependent protein kinase PKR and/or a eukaryotic translation initiation factor eIF2α.

The invention described in the second aspect specifies the protein whose phosphorylation is induced by the administration of endostatin and is the target of detection. These proteins are phosphorylated by the administration of antiangiogenic agent and therefore are thought to be involved in transducing the antiangiogenic signal. By targeting the phosphorylations of these proteins in the detection step, antiangiogenic agent can be screened with high accuracy and reliability.

In a third aspect of the present invention provided is a method for screening for an antiangiogenic agent, including: a candidate compound administration step of administering a candidate compound for the antiangiogenic agent to a vascular endothelial cell or a cultured cell derived from the vascular endothelial cell; a cell-maintaining step of maintaining the vascular endothelial cell or the cultured cell derived from the vascular endothelial cell to which the candidate compound has been administered; and a signal detection step of detecting phosphorylation of a protein phosphorylated, in which the protein whose phosphorylation is detected in the signal detection step is a double stranded RNA-dependent protein kinase PKR and/or a eukaryotic translation initiation factor eIF2α.

According to the invention described in the third aspect, a screen for an antiangiogenic agent that does not depend on protein phosphorylation induced by endostatin treatment can be carried out by detecting the phosphorylation of the double stranded RNA-dependent protein kinase PKR and/or the eukaryotic translation initiation factor eIF2α that are predicted to be also phosphorylated by other antiangiogenic promoting factors, making it possible to screen a wider range of candidate molecules efficiently.

In a fourth aspect of the method for screening for an antiangiogenic agent described in the second or third aspects, phosphorylation of Thr451 of a human double stranded RNA-dependent protein kinase PKR or phosphorylation of a corresponding amino acid residue of a double stranded RNA-dependent protein kinase PKR, and/or phosphorylation of Ser51 of a human eukaryotic translation initiation factor eIF2α or phosphorylation of a corresponding amino acid residue of a eukaryotic translation initiation factor eIF2α is detected in the signal detection step.

Here, "a corresponding amino acid residue of a double stranded RNA-dependent protein kinase PKR" means that an amino acid residue that is a phospho-acceptor site corresponding to the Thr431 of the human double stranded RNA-dependent protein kinase PKR, that is from a double stranded RNA-dependent protein kinase PKR of different species, structurally and functionally homologous to the human double stranded RNA-dependent protein kinase PKR. Similarly, "a corresponding amino acid residue of a eukaryotic translation initiation factor eIF2α" means that an amino acid residue that is a phospho-acceptor site corresponding to the Ser51 of the human eukaryotic translation initiation factor eIF2α, that is from a eukaryotic translation initiation factor eIF2α of different species, structurally and functionally homologous to the human eukaryotic translation initiation factor eIF2α.

Here, "a human double stranded RNA-dependent protein kinase PKR" and "a human eukaryotic translation initiation factor eIF2α" indicates, respectively, a double stranded RNA-dependent protein kinase PKR or a eukaryotic translation initiation factor eIF2α from humans.

The invention described in the fourth aspect specifies an amino acid residue of a protein whose phosphorylation is induced following the administration of antiangiogenic agent such as endostatin and is the target of detection. The amino acid residues of these proteins are phosphorylated by the administration of antiangiogenic agent and therefore is thought to be involved in transducing the antiangiogenic signal. By detecting the phosphorylations of the amino acid residues of these proteins in the detection step, antiangiogenic agent can be screened with high accuracy and reliability.

In a fifth aspect of the method for screening for the antiangiogenic agent described in any one of the first to fourth aspects, the vascular endothelial cell or the cultured cell derived from the vascular endothelial cell is selected from a group including capillary vessel endothelial cell, great vessel-umbilical vein endothelial cell and retina vessel endothelial cell.

The invention described in the fifth aspect specifies the cell types to be used in the screening for an antiangiogenic agent. These cells can be obtained and handled easily and are highly sensitive to endostatin, making them very useful in detecting the phosphorylation of the specific proteins responding to the antiangiogenic signaling, and in efficiently performing the screen for an antiangiogenic agent.

In a sixth aspect of the method for screening for the antiangiogenic agent described in any one of the first to fifth aspects, the signal detection step employs at least one of the detection methods selected from a group including immunoblotting using a phospho-specific antibody, autoradiography using ³²P, immuno-histochemistry using a phospho-specific antibody, gel-shift and immunoprecipitation using a specific antibody and a phospho-specific antibody.

Here, "specific antibody" refers to an antibody that can specifically bind to the specific protein to be detected in the signal detection step, regardless of the phosphorylation status of the protein. "phospho-specific antibody" refers to an antibody that can specifically bind to only the phosphorylated form of the specifc protein to be detected.

The invention described in the sixth aspect specifies the method utilized to detect the phosphorylation of the specific protein in the screening method for the antiangiogenic agent. These methods can detect protein phosphorylations efficiently with high accuracy and reliability so that the screening for an antiangiogenic agent can be carried out efficiently with high accuracy and reliability.

In a seventh aspect of the present invention provided is a method for screening for an antiangiogenic signal gene, including: an expression level alteration step of altering an expression level of a candidate antiangiogenic signal gene in a vascular endothelial cell or a cultured cell derived from the vascular endothelial cell; a cell-maintaining step of maintaining the vascular endothelial cell or the cultured cell derived from the vascular endothelial cell whose expression level of the candidate antiangiogenic gene is altered; a signal detection step of detecting phosphorylation of a protein phosphorylated by the administration of endostatin.

Here, "antiangiogenic signal gene" refers to a gene encoding a downstream effector of endostatin or its homologous antiangiogenic factors, that is either activated or inactivated in response to treatment with endostatin or its homologous antiangiogenic factors, and that constitutes a signal transduction pathway for transducing the negative regulatory signal to inhibit the expression of angiogenic genes.

In an eighth aspect of the method for screening for the antiangiogenic signal gene described in the seventh aspect, the protein phosphorylated by the administration of endostatin is a double stranded RNA-dependent protein kinase PKR and/or a eukaryotic translation initiation factor eIF2α.

In a ninth aspect of the present invention provided is a method for screening for an antiangiogenic agent, including: an expression level alteration step of altering an expression level of a candidate antiangiogenic signal gene in a vascular endothelial cell or a cultured cell derived from a vascular endothelial cell; a cell-maintaining step of maintaining the vascular endothelial cell or the cultured cell derived from the vascular endothelial cell whose expression level of the candidate antiangiogenic gene is altered; and a signal detection step of detecting phosphorylation of a protein, in which the protein whose phosphorylation is detected in the signal detection step is a double stranded RNA-dependent protein kinase PKR and/or a eukaryotic translation initiation factor eIF2α.

In a tenth aspect of the method for screening for the antiangiogenic signal gene described in the eighth or ninth aspects, phosphorylation of Thr451 of a human double stranded RNA-dependent protein kinase PKR or phosphorylation of a corresponding amino acid residue of a double stranded RNA-dependent protein kinase PKR, and/or phosphorylation of Ser51 of a human eukaryotic translation initiation factor eIF2α or phosphorylation of a corresponding amino acid residue of a eukaryotic translation initiation factor eIF2α is detected in the signal detection step.

In an eleventh aspect of the method for screening for the antiangiogenic signal gene described in any one of the seventh to tenth aspects, the expression level alteration step is an up-regulating step of overexpressing the candidate antiangiogenic signal gene in a vascular endothelial cell or in a cultured cell derived from a vascular endothelial cell, or a down-regulating step of repressing expression of the candidate antiangiogenic signal gene in a vascular endothelial cell or in a cultured cell derived from a vascular endothelial cell.

In a twelfth aspect of the method for screening for the antiangiogenic signal gene described in any one of the seventh to eleventh aspects, the vascular endothelial cell or the cultured cell derived from the vascular endothelial cell is selected from a group including capillary vessel endothelial cell, great vessel-umbilical vein endothelial cell and endothelial cell derived from retina vessel.

In a thirteenth aspect of the method for screening for the antiangiogenic signal gene described in any one of the seventh to twelfth aspects, the signal detection step for detecting the phosphorylation of the protein employs at least one of the detection methods selected from a group including immunoblotting using a phospho-specific antibody, autoradiography using ³²P, immuno-histochemistry using a phospho-specific antibody, gel-shift, and immunoprecipitation using a specific antibody and phospho-specific antibody.

The invention described in the seventh to thirteenth aspects is derived from the invention described in the first to sixth aspects with the aim of screening for an antiangiogenic signal gene. As such, the invention according to the seventh to thirteenth aspects, imparts similar effect as the invention described in the first to sixth aspects.

Especially, in regard to the invention described in the eleventh aspect, the induced antiangiogenic signaling is detected in the signal detection step by up-regulating or down-regulating the expression of a candidate antiangiogenic signal gene in the expression level alteration step. By employing this method, it becomes possible to screen for genes that function to enhance the angiogenic signaling as well as for genes that function to inhibit the angiogenic signaling.

### Effects of the Invention

The screening method for an antiangiogenic agent described in the present invention is based on detecting phosphorylation of proteins whose phosphorylation is induced by the administration of endostatin, and as such, can be used to efficiently screen a large number of candidate compounds.

The screening method for an antiangiogenic agent described in the present invention detects the phosphorylation of the double stranded RNA-dependent protein kinase PKR and/or eukaryotic translation initiation factor eIF2α that are predicted to be phosphorylated not only as the result of endostatin administration but also as the result of administering other antiangiogenic factors. Therefore, this method can screen a wide range of candidate compounds very efficiently.

Similarly, the screening method for an antiangiogenic signal gene described in the present invention is based on detecting the phosphorylation of the proteins whose phosphorylations are induced by the administration of endostatin, and as such, can be used to efficiently screen a large number of candidate genes.

The screening method for an antiangiogenic agent described in the present invention detects the phosphorylation of the double stranded RNA-dependent protein kinase PKR and/or eukaryotic translation initiation factor eIF2α that are predicted to be phosphorylated not only as the result of endostatin administration but also as the result of administering other antiangiogenic factors. Therefore, this method can screen a wide range of candidate genes very efficiently.

### BRIEF DESCRIPTION OF THE DRAWINGS

Fig 1: Is a graph showing results of the antibody microarray experiment according to Example 1 of the present invention;
Fig 2: Is a graph showing results of the quantitative western blotting experiment according to Example 2 of the present invention;
Fig 3: Is an image showing results of the western blotting experiment according to Example 3 of the present invention;
Fig 4: Is an image showing the fluorescent antibody experiment according to Example 4 of the present invention;
Fig 5: Is a graph showing expression levels of the PKR gene;
Fig 6: Is a graph showing expression levels of the ID₁ gene;
Fig 7: Is a graph showing expression levels of the ID₃ gene;
Fig 8: Is a graph showing expression levels of the integrinαv gene;
Fig 9: Is a graph showing expression levels of the Flt gene;
Fig 10: Is a graph showing expression levels of the Ephrin A1 gene; and
Fig 11: Is a graph showing results of the real-time quantitative RT-PCR experiment according to Example 7 of the present invention.

### PREFERRED MODE FOR CARRYING OUT THE INVENTION

Preferred mode for carrying out the present invention is described in detail below.

### Screening method for an antiangiogenic agent

Screening method for an antiangiogenic agent of the present invention is described below.

Screening method for an antiangiogenic agent of the present invention includes a candidate compound administration step of administering a candidate compound for the antiangiogenic agent to a vascular endothelial cell or a cultured cell derived from the vascular endothelial cell; a cell-maintaining step of maintaining the vascular endothelial cell or the cultured cell derived from the vascular endothelial cell to which the candidate compound has been administered; and a signal detection step of detecting the phosphorylation of a protein phosphorylated by the administration of endostatin.

Screening method for an antiangiogenic agent of the present invention also includes a candidate compound administration step of administering a candidate compound for the antiangiogenic agent to a vascular endothelial cell or a cultured cell derived from the vascular endothelial cell; a cell-maintaining step of maintaining the vascular endothelial cell or the cultured cell derived from the vascular endothelial cell to which the candidate compound has been administered; and a signal detection step of detecting the phosphorylation of a double stranded RNA-dependent protein kinase PKR and/or a eukaryotic translation initiation factor eIF2α.

### [Candidate compound administration step]

In the candidate compound administration step, the candidate compound for the antiangiogenic agent is administered to the vascular endothelial cell or the cultured cell derived from the vascular endothelial cell. Candidate compound can be administered alone or in a combination of two or more compounds.

### (Candidate compound)

There are no restrictions on the nature of the candidate compound. It can be a low molecular compound or a high molecular compound. As an example of low molecular compound, one can use numerous compounds manufactured in the process of drug development. These compounds can be subjected to the screen for an antiangiogenic agent as a library of candidate compounds for the antiangiogenic agent. As an example of high molecular compound, one can envisage proteins, and of excreted protein libraries expressing specific cDNAs isolated from the cells involved in the inhibition of angiogenesis, or alternatively, of protein libraries expressing excreted proteins whose expression level is enhanced upon the stimulation by specific signals involved in the inhibition of angiogenesis. These could be subjected to the screening for an antiangiogenic agent as well.

Among these, the use of low molecular compounds is most preferable from the standpoint of large-scale synthesis and administration to patients, once an effect as an antiangiogenic agent has been discovered.

### (A vascular endothelial cell or a cultured cell derived from a vascular endothelial cell)

There are no restrictions on the type of the vascular endothelial cell or the cultured cell derived from the vascular endothelial cell that is used in the screen for an antiangiogenic agent. Any type of cells can be used. In particular, a human vascular endothelial cell or a cultured cell derived from a human vascular endothelial cell, such as skin capillary blood vessel endothelial cell, umbilical vein endothelial cell, endothelial cell isolated from tumors, and retina vessel endothelial cell and cell line derived therefrom; mouse vascular endothelial cell or cultured cell derived therefrom, such as kidney endothelial cell line, lymphatic node endothelial cell line, pancreatic islet endothelial cell line, and ES cell derived endothelial cell line; rat vascular endothelial cell or cultured cell derived therefrom, such as lung artery derived primary endothelial cell or cell line derived therefrom, aorta derived primary endothelial cell, and endothelial cells isolated from tumors, can be used. Among these, human capillary blood vessel endothelial cell, endothelial cell isolated from tumors, umbilical vein endothelial cell, and retina vessel endothelial cell are preferred considering the ease with which they can be obtained and handled, the accuracy with which they reflect the pathology of tumor angiogenesis and retina vessel angiogenesis, and the sensitivity to endostatin.

### (Administration method)

In the screen for an antiangiogenic agent, there are no restrictions on the methods of administering the candidate compound and any methods that are publicly available can be used. For instance, one can dissolve the candidate compound into a solution, add it to the culture medium for culturing the vascular endothelial cell or the cultured cell derived from the vascular endothelial cell, and continue the culturing of cells in the presence of the compound. One can also add relatively high concentrated form of the compound solution to the medium, and replace the medium supplemented with the compound within a short period of time. An appropriate administering method should be chosen depending on the predicted nature of the compound to be tested in the screen as an antiangiogenic agent.

### [Cell-maintaining step]

In the cell-maintaining step, the vascular endothelial cell or the cultured cell derived from the vascular endothelial cell to which the candidate compound has been administered is maintained. During the cell-maintaining period, the phosphorylation of proteins that are phosphorylated in response to endostatin administration as well as the phosphorylation of double stranded RNA-dependent protein kinase PKR and/or eukaryotic translation initiation factor eIF2α are enhanced as the result of the antiangiogenic signal transduction elicited by the administration of the candidate compound, thereby increasing the sensitivity of detecting the protein phosphorylation in the signal detection step below.

There are no restrictions on the way in which the cells are maintained. The cells can be maintained under regular culture conditions. In particular, cells can be maintained in the presence of 2% to 17% CO₂, and within the temperature range of 33°C to 38°C. Appropriate culture medium should be chosen for instance, from DMEM, MEM, RPMI and HAM medium, depending on the cell type.

Regarding the length of the time the cells should be maintained in the cell-maintaining step, it should preferably be no less than 1 minute and no greater than 8 hours, more preferably be no less than 3 minutes and no greater than 6 hours, and most preferably no less than 10 minutes and no greater than 60 minutes. If the time is less than one minute, there is a chance that the protein phosphorylation of interest still has not proceeded fully inside the cell, severely compromising the detection sensitivity in the signal detection step. On the other hand, if the time is greater than 8 hours, the screening efficiency is significantly reduced by taking too much time to perform the screening procedure. In addition, it could lead to desensitization of the receptor and other factors, resulting in the reduction of protein phosphorylation of interest.

### [Signal detection step]

In the signal detection step, endostatin administration induced protein phosphorylation is detected. By going through the signal detection step, one can identify a compound that elicits antiangiogenic signaling among the candidate compounds.

In the signal detection step, not only endostatin administration induced protein phosphorylation is detected. The phosphorylation of double stranded RNA-dependent protein kinase PKR and/or eukaryotic translation initiation factor eIF2α can be detected independently of endostatin administration induced phosphorylation. Thus, by going through the signal detection step, one can efficiently screen a wide range of candidate compounds for an antiangiogenic agent.

### (Proteins that are phosphorylated by the administration of endostatin)

In the present invention, activation of antiangiogenic signaling is measured by detecting protein phosphorylation induced by endostatin administration. There are no restrictions on the proteins that are phosphorylated by the administration of endostatin, and any antiangiogenic signaling component involved in the regulation of angiogenesis related gene expression that functions down stream of endostatin can be used.

As an example of such a factor mediating the antiangiogenic signaling, one can utilize the double stranded RNA-dependent protein kinase PKR and/or the eukaryotic translation initiation factor eIF2α. With respect to the actual phosphorylation sites, one can utilize Thr 451 or Thr 446 of the human double stranded RNA-dependent protein kinase PKR, and/or the Ser 51 of human eukaryotic translation initiation factor eIF2α. In addition, one can also utilize the amino acid residues that correspond to those phosphorylated in human proteins in a double stranded RNA-dependent protein kinase PKR or a eukaryotic translation initiation factor eIF2α from other species that are structurally as well as functionally homologous to the human proteins. The preferable phosphorylation site in the double stranded RNA-dependent protein kinase PKR is Thr 451, while the preferable site in the eukaryotic translation initiation factor eIF2α is Ser 51.

By detecting the site-specific phosphorylation of the antiangiogenic signaling factors, it becomes possible to screen for an antiangiogenic agent with high accuracy and reliability.

### (Double stranded RNA-dependent protein kinase PKR and eukaryotic translation initiation factor eIF2α)

In the signal detection step, one can detect the activation of the antiangiogenic signaling by measuring the phosphorylation of double stranded RNA-dependent protein kinase PKR and/or eukaryotic translation initiation factor eIF2α, independently of endostatin induced protein phosphorylation. By detecting the phosphorylation of double stranded RNA-dependent protein kinase PKR and/or eukaryotic translation initiation factor eIF2α, one can screen for an antiangiogenic agent with high accuracy and reliability. The preferable phosphorylation site in the double stranded RNA-dependent protein kinase PKR is Thr 451, and the preferable site in the eukaryotic translation initiation factor eIF2α is Ser 51.

Traditionally, the measurement of the changes in the expression level of genes involved in angiogenesis has been very inaccurate due to the issue of non-specific changes. The inventors of the present invention focused on the changes in the level of phosphorylation in a protein as a benchmark reflecting the specific change in the level of gene expression, and used the "phospho-specific antibody microarray" technology to systematically identify the proteins that are phosphorylated and to examine the extent to which these proteins are phosphorylated. The use of the "phospho-specific antibody microarray" technology allows accurate measurement of the changes in the amount of phosphorylated proteins that play important roles in angiogenesis. After examining the data on the extent of phosphorylation of these proteins, the inventors of the present invention discovered that the phosphorylation of double stranded RNA-dependent protein kinase PKR and/or eukaryotic translation initiation factor eIF2α, correlated tightly with the inhibition of expression of the genes involved in angiogenesis in human vascular endothelial cells.

The antiangiogenic agents that induce phosphorylation of double stranded RNA-dependent protein kinase PKR and/or eukaryotic translation initiation factor eIF2α, include rifampicin, endostatin and angiostatin.

As the inventors of the present invention demonstrate in the Examples below, the antiangiogenic signal elicited by endostatin, etc., causes an increase in the amount of mRNA that encodes double stranded RNA-dependent protein kinase PKR, which in turn causes an increase in the amount of PKR protein expressed. Therefore, during the signal detection step, not only does the level of PKR phosphorylation increases as a result of antiangiogenic signaling, but the absolute amount of PKR protein to be phosphorylated also increases, further contributing to the ease with which the phosphorylated PKR is detected in the signal detection step.

### (Method for detecting phosphorylation)

There are no restrictions on the method for detecting the endostatin administration induced protein phosphorylation, and the phosphorylations of double stranded RNA-dependent protein kinase PKR and/or eukaryotic translation initiation factor eIF2α. Any of the publicly available methods that are being widely used as the methods for detecting phosphorylation can be used. For example, immunoblotting using phospho-specific antibody, ³²P autoradiography, immuno-histochemistry using phospho-specific antibody, gel-shift method, and immunoprecipitation using specific antibody and phospho-specific antibody, can all be used. Among these, immunoblotting and immuno-histochemistry are preferable methods considering the ease of experimental procedure as well as the ease of detection.

The above detection method is preferably performed with widely available method. For example, in the case of immunoblotting method, total cell lysate is first obtained after the cells are administered with the candidate compound and maintained as specified above. The proteins are denatured in the presence of detergent, if necessary, and separated by electrophoresis. The separated proteins are then transferred to a protein binding membrane such as a nylon membrane. The phosphorylated PKR is detected by reacting a phospho-specific anti-PKR antibody as the primary antibody to the membrane.

In the ³²P autoradiography method, the candidate compound is administered to the cells after the cells were incubated withy-³²P-ATP, to generate ³²P labeled phospho-PKR in the cells. Subsequently, using the same method described for immunoblotting method, the proteins are separated by electrophoresis, transferred to a protein binding membrane, and then phospho-PKR labeled with ³²P is detected.

In the immuno-histochemistry method using a phospho-specific antibody, the cells treated with the candidate compound are fixed, for example with formaldehyde etc., and further treated with detergent if necessary. Phospho specific anti-PKR antibody is then added to the cells, followed by fluorescence-labeled secondary antibody against the primary antibody. Phospho-PKR can then be detected under a fluorescence microscope.

In the gel-shift method, the cells treated with the candidate compound are processed using the same method as described in immunoblotting method, and the proteins are separated by electrophoresis and transferred to a protein binding membrane such as a nylon membrane. PKR can then be detected by reacting anti-PKR antibody as the primary antibody to the membrane. By detecting the PKR band, the shift in the mobility of the band due to the phosphorylation can be visualized, and the phosphorylation can be detected.

In the immunoprecipitation method using specific antibody and phospho-specific antibody, the total cell lysate is first prepared from cells treated with the candidate compound, and the proteins in the lysate are reacted with specific antibody or phospho-specific antibody. Subsequently, precipitated reactants are separated by electrophoresis and transferred to a protein binding membrane. Phosphorylated protein can then be detected by reacting the membrane with phospho specific antibody or specific antibody as the primary antibodies.

Using the screening method for an antiangiogenic agent of the present invention, one can screen for an antiangiogenic agent by detecting the protein phosphorylation induced by endostatin administration or by detecting the phosphorylation of double stranded RNA-dependent protein kinase PKR and/or eukaryotic translation initiation factor eIF2α.

As described above, the target protein is rapidly phosphorylated following the treatment of the vascular endothelial cell or the cultured cell derived from the vascular endothelial cell with the candidate antiangiogenic agent. Therefore, the screening can be carried out efficiently even with a large number of candidate compounds.

### The screening method for an antiangiogenic signal gene

The method for screening for an antiangiogenic signal gene of the present invention is described in detail below. Detailed explanations are omitted in some areas where the aspects of the method for screening for an antiangiogenic signal gene are identical to the method for screening for an antiangiogenic agent described above.

The method for screening for an antiangiogenic signal gene of the present invention includes an expression level alteration step of altering the expression level of a candidate antiangiogenic signal gene in a vascular endothelial cell or a cultured cell derived from the vascular endothelial cell; a cell-maintaining step of maintaining the vascular endothelial cell or the cultured cell derived from the vascular endothelial cell whose expression level of the candidate antiangiogenic gene is altered; and a signal detection step of detecting phosphorylation of a protein phosphorylated by the administration of endostatin.

The method for screening for an antiangiogenic signal gene of the present invention includes an expression level alteration step of altering the expression level of a candidate antiangiogenic signal gene in a vascular endothelial cell or a cultured cell derived from the vascular endothelial cell; a cell-maintaining step of maintaining the vascular endothelial cell or the cultured cell derived from the vascular endothelial cell whose expression level of the candidate antiangiogenic gene is altered; and a signal detection step of detecting phosphorylation of a protein, in which the phosphorylated protein detected in the signal detection step is a double stranded RNA-dependent protein kinase PKR and/or a eukaryotic translation initiation factor eIF2α.

### [Expression level alteration step]

In the expression level alteration step, the expression level of a candidate antiangiogenic signal gene is altered. If the candidate gene whose expression level has been altered is an antiangiogenic signal gene that encodes a factor that enhances or inhibits the antiangiogenic signal, protein phosphorylation induced by endostatin administration or phosphorylation of double stranded RNA-dependent protein kinase PKR and/or eukaryotic translation initiation factor eIF2α is enhanced. Antiangiogenic signal gene can be screened either by detecting the protein phosphorylation induced by endostatin administration or by detecting the phosphorylation of double stranded RNA-dependent protein kinase PKR and/or eukaryotic translation initiation factor eIF2α, in a condition where the expression level of the candidate gene is altered.

### (Candidate gene)

There are no restrictions on the candidate gene for the antiangiogenic signaling gene and it can be any genes such as, for example, those that are being expressed in vascular endothelial cells. These genes can be obtained in the form of cDNA libraries made from various vascular endothelial cell sources. The method for producing an endothelial cDNA library can be any method that is currently being widely used, for example, purifying mRNA from vascular endothelial cells, converting mRNA into cDNA by reverse transcription, treating with restriction enzymes as required, and finally sub-cloning into a vector.

### (Up-regulation step)

The expression level alteration step can be an up-regulation step in which the candidate gene for an antiangiogenic signal gene is over-expressed. When expression level alteration step is designed as an up-regulation step, the screen for an antiangiogenic signal gene of the present invention will identify antiangiogenic signal genes that function to enhance the antiangiogenic signal.

There are no restrictions on the methods for overexpressing candidate genes for antiangiogenic signal gene in a vascular endothelial cell or a cultured cell derived from a vascular endothelial cell, and can be any of the publicly available methods that are being widely used today. Examples include cloning the candidate gene into a plasmid, cosmid, or viral vector and transfecting it into the cells.

### (Down-regulation step)

The expression level alteration step can be a down-regulation step in which the expression of candidate gene for an antiangiogenic signal gene is inhibited. When expression level alteration step is designed as a down-regulation step, the screen for an antiangiogenic signal gene of the present invention will identify antiangiogenic signal genes that function to inhibit the antiangiogenic signal.

There are no restrictions on the methods for inhibiting the expression of candidate genes for antiangiogenic signal gene in a vascular endothelial cell or a cultured cell derived from a vascular endothelial cell, and can be any methods, for example, RNA interference.

Using the screening method for an antiangiogenic signal gene of the present invention, one can screen for antiangiogenic signal genes by detecting the protein phosphorylation induced by endostatin administration. The target protein is rapidly phosphorylated following the alteration of the expression of an antiangiogenic signal gene in a vascular endothelial cell or a cultured cell derived from a vascular endothelial cell. Therefore, the screening can be carried out efficiently even with a large number of candidate genes.

The screening method for an antiangiogenic agent described in the present invention also can efficiently screen a wide range of candidate compounds because the screen for an antiangiogenic agent does not solely depend on the protein phosphorylation induced by endostatin administration but also on detecting the phosphorylation of the double stranded RNA-dependent protein kinase PKR and/or eukaryotic translation initiation factor eIF2α that are predicted to be phosphorylated by other antiangiogenic promoting factors.

### EXAMPLES

The Examples of the present invention are described in detail below, with references to figures. This invention is in no way restricted by the Examples that follow.

### Example 1

Rifampicin is known to have an antiangiogenic activity similar to that of endostatin. Identifying proteins that are phosphorylated in response to administering rifampicin to vascular endothelial cell therefore is expected to lead to identifying candidate proteins that are also phosphorylated by administering endostatin to vascular endothelial cell.

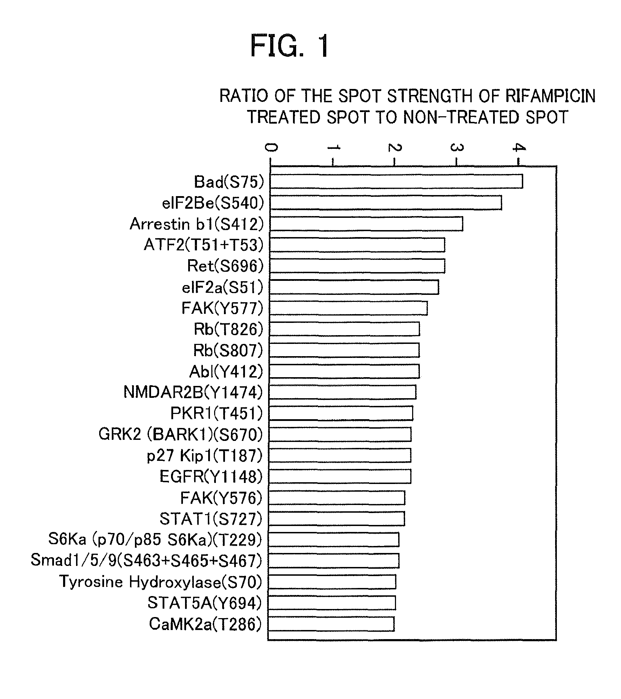

To screen for proteins whose phosphorylation is induced by rifampicin administration, the antibody microarray technology was used to identify proteins that are specifically phosphorylated after rifampicin treatment. Rat aorta endothelial cells (rAEC) were cultured in DMEM supplemented with 10% Bovine Fetal Serum with or without rifampicin (40 µg/ml) at 37°C for 10 minutes, and the total cell extracts were subjected to analysis by the "Kinex^{™} antibody microarray" (Kinexus Bioinformatics) with 630 characterized antibodies to signaling proteins. Figure 1 shows the results of the experiment. The proteins whose phosphorylation levels markedly increased by rifampicin treatment are shown together with the extent of the increase.

From Figure 1, it can be seen that a number of proteins exhibit significantly increased phosphorylation following the ripampicin treatment of Rat aorta endothelial cells, including Bad, eIF2α and PKR1.

### Example 2

In the antibody microarray technology, there is the possibility that false positives may occur due to antibody cross-reactivity and blocked epitopes in protein complexes. Quantitative western blotting was therefore performed on the proteins identified as positives in Example 1 to confirm the increase in the phosphorylation level.

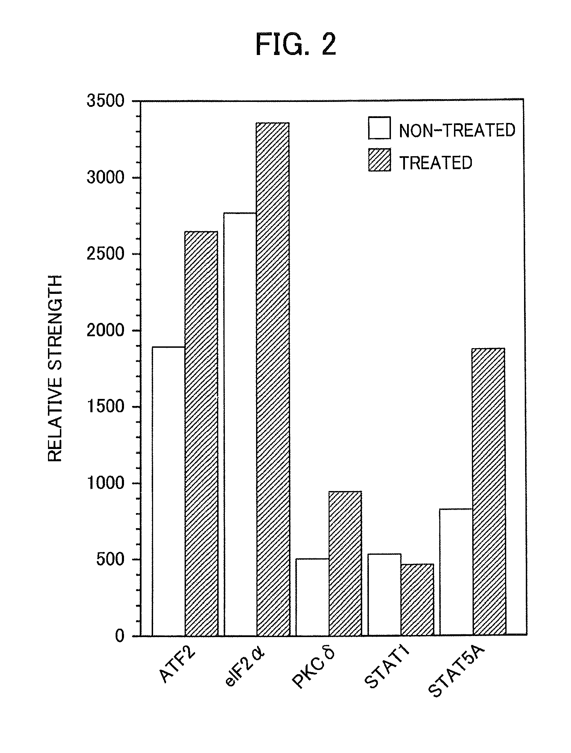

Rat aorta endothelial cells (rAEC) were cultured in DMEM supplemented with 10% Bovine Fetal Serum with or without rifampicin (40 µg/ml) at 37°C for 10 minutes, and the amount of respective phosphorylated proteins in the total cell extracts were determined in a quantitative western blotting experiment using the phospho-specific antibodies against ATF2, eIF2α, PKCδ, STAT1 and STAT5A. The results are shown in Figure 2.

Figure 2 shows that among the proteins that were scored positive in the antibody microarray of Example 1, the phosphorylations of ATF2, eIF2α, PKCδ and STAT5A at least were confirmed to also increase in the quantitative western blotting experiment. On the other hand, the increase of phosphorylation of STAT1 was not confirmed in the quantitative western blotting experiment, suggesting that the STAT1 result obtained in the antibody microarray experiment of Example 1 is not reproducible.

### Example 3

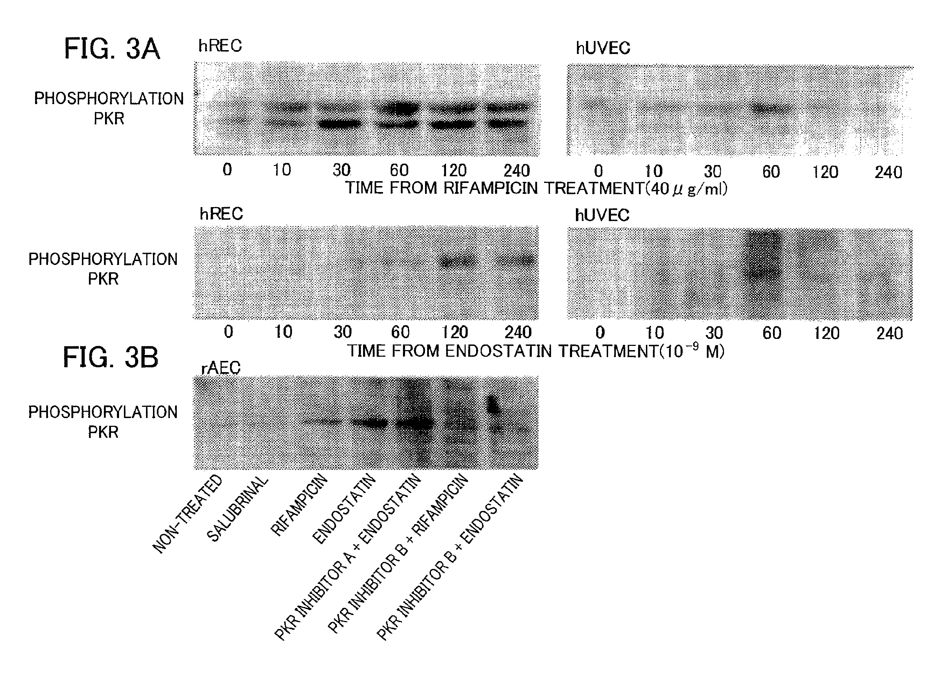

Human retina vessel endothelial cells (hREC) or human umbilical vein endothelial cells (hUVEC) cultured in HamF12K medium supplemented with 10% bovine fetal serum and growth factors were treated with either 40 µg/ml rifampicin or 1.0X10⁻⁹ M endostatin, and maintained at 37°C for 10 to 240 minutes. Total cell extracts from the maintained cells were then subjected to western blotting using phospho-specific anti-PKR antibody to examine the time course of PKR phosphorylation. The results are shown in Figure 3(a).

Similarly, mouse aorta endothelial cells (rAEC) were treated respectively with salubrinal (20 µM), rifampicin (40 µg/ml), or endostatin (1.0X10⁻⁹ M); PKR inhibitor A (2-aminopurine, 10 mM) followed by endostatin (1.0X10⁻⁹ M); PKR inhibitor B (8-(imidazole-4-ylmethylene)-6H-azolidino [5,4-g] benzodiazole-7-one, 0.3 µM) followed by rifampicin (40 µg/ml); and PKR inhibitor B (same as above, 0.3 µM) followed by endostatin (1.0X10⁻⁹ M); and maintained at 37°C for 4 or 8 hours. Total cell extracts from respective samples were subjected to western blotting analysis using phospho-specific anti-PKR antibody to determine the extent of PKR phosphorylation. The results are shown in Figure 3(b).

As can be seen in Figure 3(a), the phosphorylation of PKR in human retina endothelial cells and human umbilical vein endothelial cells is enhanced in response to rifampicin or endostatin treatment. This suggests that the phosphorylation of PKR by rifampicin treatment identified in Example 1 is specific. As shown in Figure 3(b), rifampicin or endostatin induced phosphorylation of PKR in rat aorta derived primary endothelial cells. However, when the cells were pre-treated with specific PKR inhibitor (8-(imidazole-4-ylmethylene)-6H-azolidino [5,4-g] benzodiazole-7-one), rifampicin or endostatin did not induce PKR phosphorylation.

### Example 4

Human umbilical endothelial cells cultured in HamF12K were treated with 40 µg/ml of rifampicin or 1.0X10⁻⁹ M of endostatin, and maintained at 37°C for 30 minutes. Cells were then stained using phospho-specific anti-PKR antibody as the primary antibody, and the presence of phosphorylated PKR was examined in the cells.

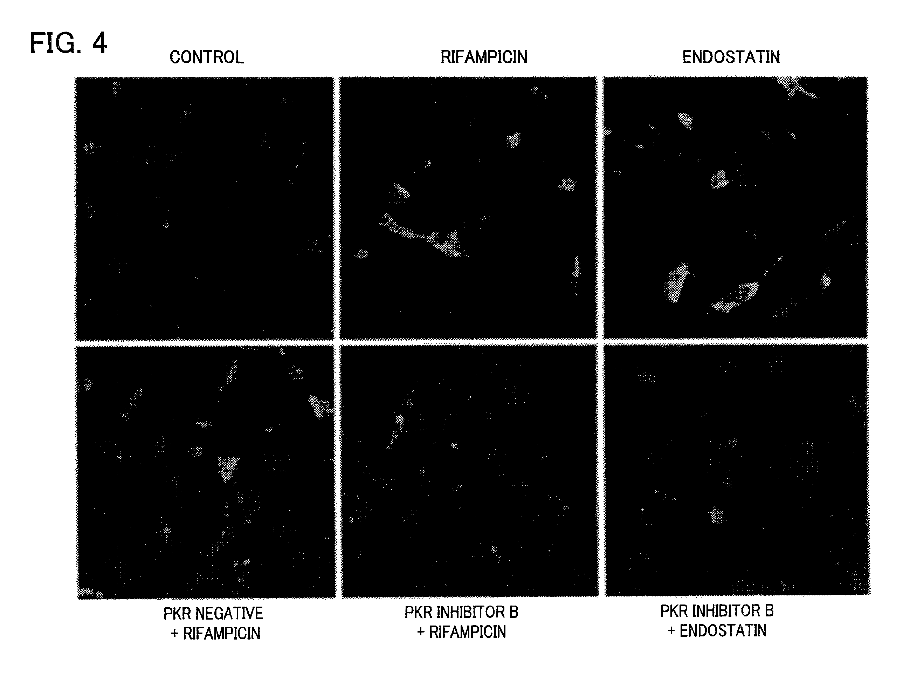

Similar experiments were also performed in human umbilical vein endothelial cells pre-treated with structural analog of PKR inhibitor (PKR negative) and then treated with 40 µg/ml rifampicin, as well as in human umbilical vein endothelial cells pre-treated with PKR inhibitor B (8-(imidazole-4-ylmethylene)-6H-azolidino [5,4-g] benzodiazole-7-one, 0.3µM) and then treated with 40 µg/ml rifampicin or 1.0X10⁻⁹ M endostatin, respectively. The results are shown in Figure 4.

As can be seen in Figure 4, rifampicin or endostatin treatment of human umbilical vein endothelial cells induced enhancement of PKR phosphorylation in the cell. This phosphorylation of PKR after rifampicin or endostatin treatment did not occur in human umbilical vein endothelial cells pre-treated with specific PKR inhibitor.

### Example 5

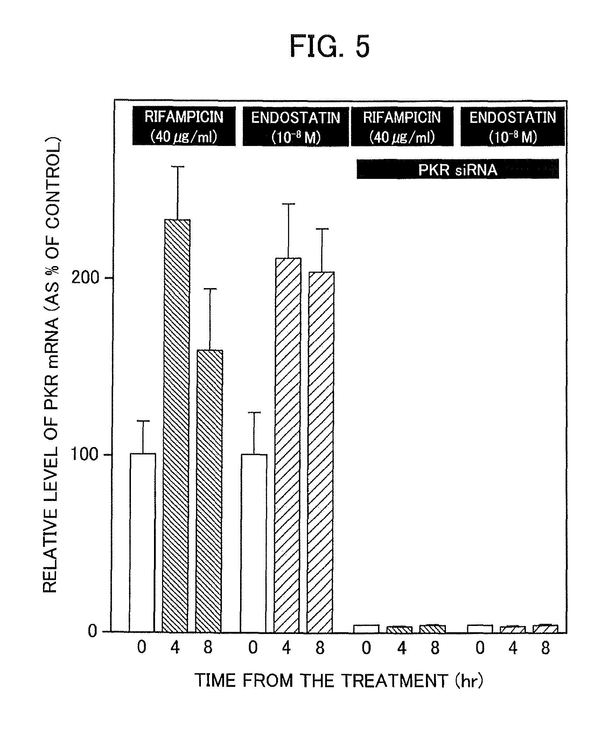

Human umbilical vein endothelial cells cultured in HamF12K medium were treated with 40 µg/ml rifampicin or 1.0X10⁻⁸ M endostatin, and maintained at 37°C for 4 to 8 hours. Subsequently, total cell extracts were subjected to real-time quantitative RT-PCR using TaqMan probe for PKR mRNA, and the amount of PKR mRNA was quantified. Similar processes were repeated for human umbilical vein endothelial cell samples that were transfected with PKR siRNA prior to rifampicin or endostatin treatment. The results are shown in Figure 5.

Figure 5 shows that PKR expression is increased in human umbilical vein endothelial cells treated with rifampicin or endostatin.

These results suggest that the expression level of PKR protein increases by rifampicin or endostatin administration. Antiangiogenic signal induces an increase in the expression level of PKR protein that then becomes the target of phosphorylation, which in turn increases the amount of phosphorylated PKR protein. By detecting the phosphorylation of PKR in the signal detection step, the screen for an antiangiogenic agent or for an antiangiogenic signal gene can be performed with great sensitivity.

### Example 6

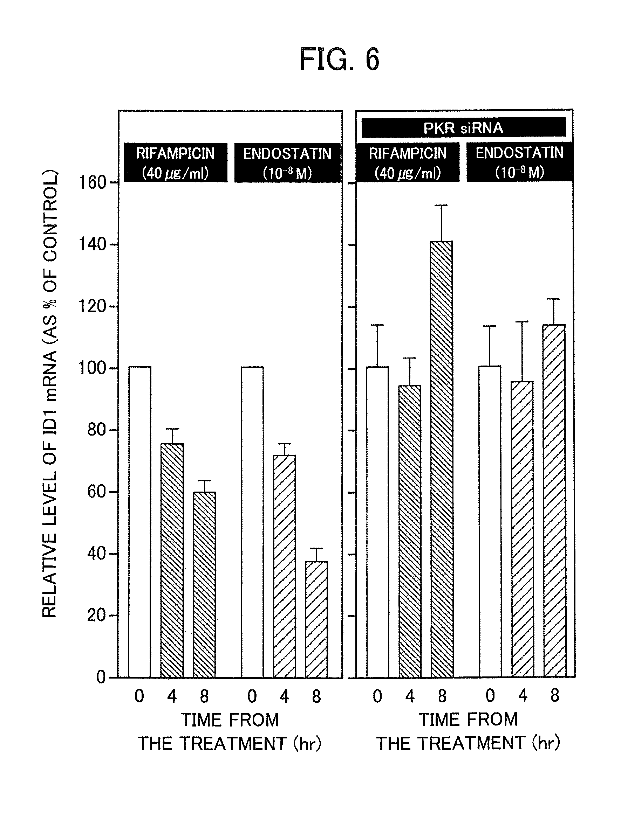

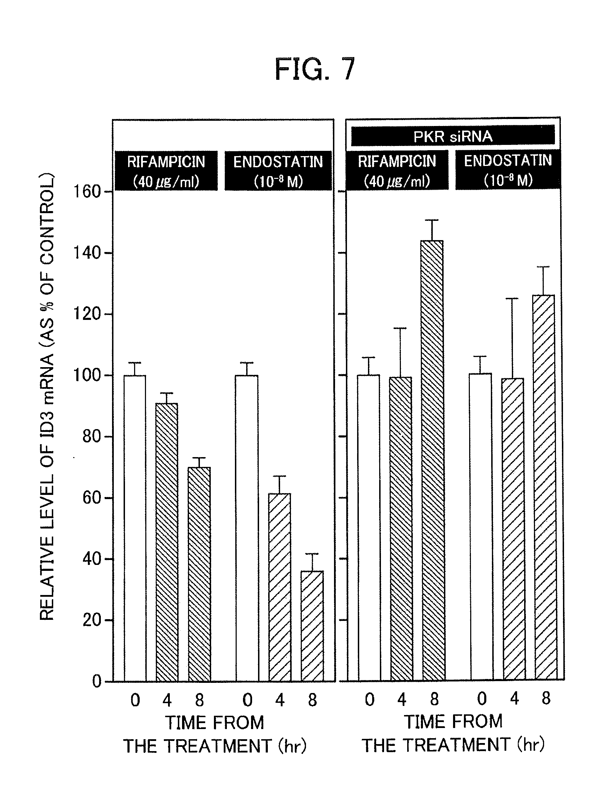

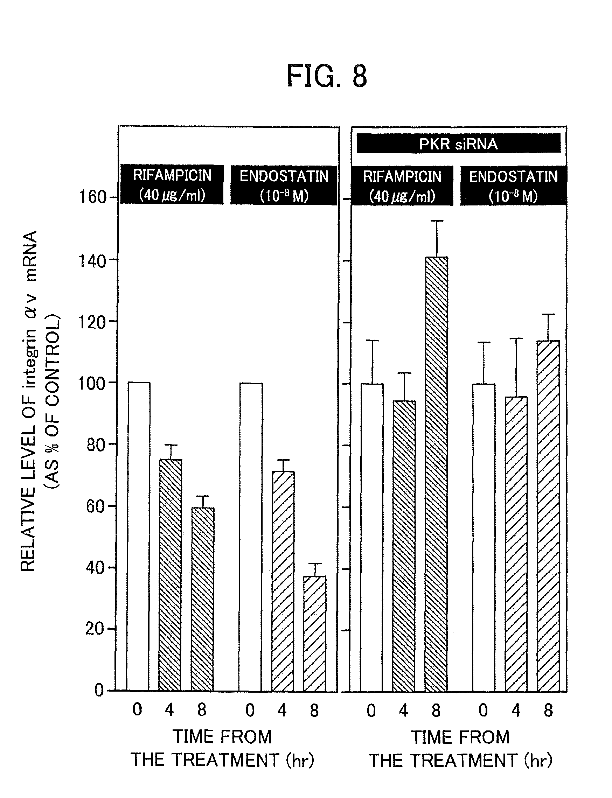

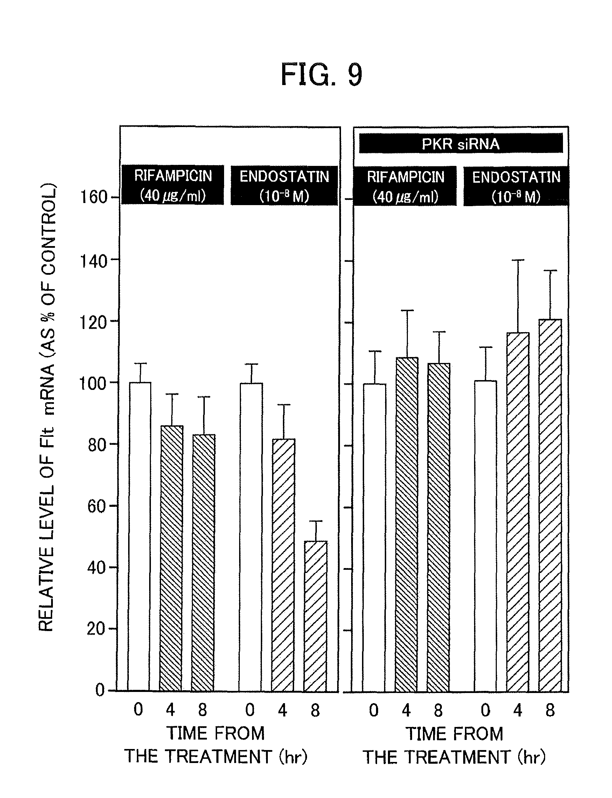

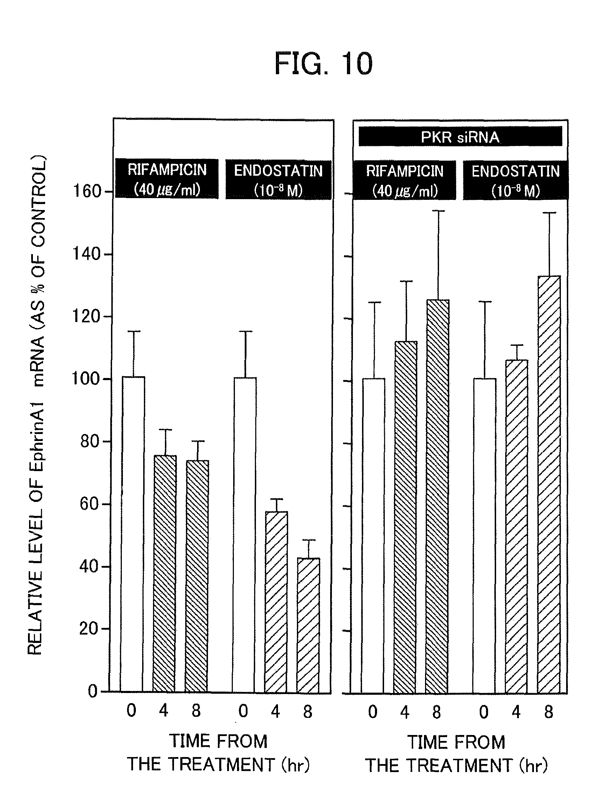

Human umbilical vein endothelial cells cultured in HamF12K medium were treated with 40 µg/ml rifampicin or 1.0X10⁻⁸ M endostatin, and maintained at 37°C for 4 to 8 hours. Subsequently, total cell extracts were subjected to real-time quantitative RT-PCR using TaqMan probe for ID₁ gene, ID₃ gene, integrin_{αv} gene, Flt gene and Ephrin A1 gene, respectively, whose expression are all known to be repressed by endostatin. After establishing the quantification condition, the amount of mRNA for these genes were quantified. Similar processes were repeated for human umbilical vein endothelial cell samples that were transfected with PKR siRNA prior to rifampicin or endostatin treatment. The results are shown in Figures 6 to 10.

Figures 6 to 10 show that the expression level of ID₁ gene, ID₃ gene, integrin_{αv} gene, Flt gene and Ephrin A1 gene are all decreased after rifampicin or endostatin treatment. In samples where PKR expression was knocked out by PKR siRNA treatment, the decrease of expression of these genes did not occur after rifampicin or endostatin treatment, suggesting that the decrease of expression of these genes by rifampicin or endostatin treatment is dependent on PKR.

### Example 7

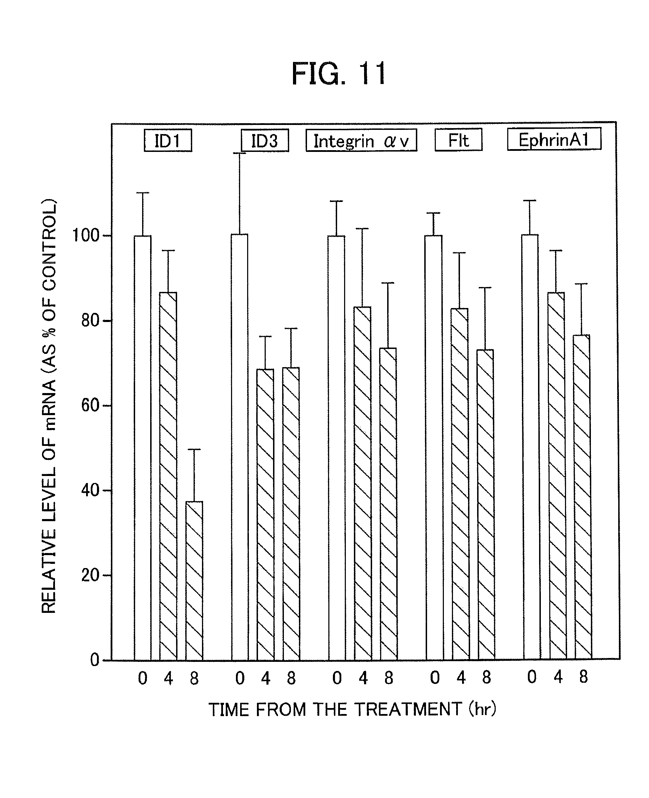

The effect of salubrinal, a potent inhibitor of eIF2α dephosphorylation, on the expression of genes that are repressed in response to endostatin treatment was examined by real-time quantitative RT-PCR. Human umbilical vein endothelial cells cultured in HamF12K medium were treated with 20 µM salubrinal, and maintained at 37°C for 4 to 8 hours. Subsequently, total cell extracts were subjected to real-time quantitative RT-PCR using TaqMan probe for ID₁ gene, ID₃ gene, integrin_{αv} gene, Flt gene and Ephrin A1 gene, respectively. After establishing the quantification condition, the amount of mRNA for these genes were quantified. The results are shown in Figure 11.

Figure 11 shows that the inhibition of the dephosphorylation of eIF2α, and thus the activation of the eIF2α activity mediated by salubrinal, leads to a decrease in the expression levels of mRNAs for ID₁ gene, ID₃ gene, integrin_{αv} gene, Flt gene and Ephrin A1 gene. This result demonstrates that the decrease in gene expression of these genes by rifampicin or endostatin treatment, can be mimicked by inhibiting the dephosphorylation of eIF2α.

## Claims

1. A method for screening for an antiangiogenic agent, comprising: a candidate compound administration step of administering a candidate compound for the antiangiogenic agent to a vascular endothelial cell or a cultured cell derived from the vascular endothelial cell;
a cell-maintaining step of maintaining the vascular endothelial cell or the cultured cell derived from the vascular endothelial cell to which the candidate compound has been administered; and
a signal detection step of detecting phosphorylation of a protein phosphorylated by administration of endostatin.

2. The method for screening for the antiangiogenic agent recited in claim 1, wherein the protein phosphorylated by administration of endostatin is at least one of a double stranded RNA-dependent protein kinase PKR and a eukaryotic translation initiation factor eIF2α.

3. A method for screening for an antiangiogenic agent, comprising: a candidate compound administration step of administering a candidate compound for the antiangiogenic agent to a vascular endothelial cell or a cultured cell derived from the vascular endothelial cell;
a cell-maintaining step of maintaining the vascular endothelial cell or the cultured cell derived from the vascular endothelial cell to which the candidate compound has been administered; and
a signal detection step of detecting phosphorylation of a protein,
wherein the protein whose phosphorylation is detected in the signal detection step is at least one of a double stranded RNA-dependent protein kinase PKR and a eukaryotic translation initiation factor eIF2α.

4. The method for screening for the antiangiogenic agent recited in claim 2 or 3, wherein at least one of phosphorylation of Thr451 of a human double stranded RNA-dependent protein kinase PKR or phosphorylation of a corresponding amino acid residue of a double stranded RNA-dependent protein kinase PKR, and phosphorylation of Ser51 of a human eukaryotic translation initiation factor eIF2α or phosphorylation of a corresponding amino acid residue of a eukaryotic translation initiation factor eIF2α, is detected in the signal detection step.

5. The method for screening for the antiangiogenic agent recited in any one of claims 1 to 4, wherein the vascular endothelial cell or the cultured cell derived from the vascular endothelial cell is selected from a group comprising capillary vessel endothelial cells, great vessel-umbilical vein endothelial cells and retina vessel endothelial cells.

6. The method for screening for the antiangiogenic agent recited in any one of claims 1 to 5, wherein the signal detection step employs at least one of detection methods selected from a group comprising immunoblotting using a phospho-specific antibody, autoradiography using ³²P, immuno-histochemistry using a phospho-specific antibody, gel-shift, and immunoprecipitation using a specific antibody and a phospho-specific antibody.

7. A method for screening for an antiangiogenic signal gene, comprising: an expression level alteration step of altering an expression level of a candidate antiangiogenic signal gene in a vascular endothelial cell or a cultured cell derived from the vascular endothelial cell;
a cell-maintaining step of maintaining the vascular endothelial cell or the cultured cell derived from the vascular endothelial cell whose expression level of the candidate antiangiogenic gene is altered;
a signal detection step of detecting phosphorylation of a protein phosphorylated by administration of endostatin.

8. The method for screening for the antiangiogenic signal gene recited in claim 7, wherein the protein phosphorylated by administration of endostatin is at least one of a double stranded RNA-dependent protein kinase PKR and a eukaryotic translation initiation factor eIF2α.

9. A method for screening for an antiangiogenic agent, comprising: an expression level alteration step of altering an expression level of a candidate antiangiogenic signal gene in a vascular endothelial cell or a cultured cell derived from the vascular endothelial cell;
a cell-maintaining step of maintaining the vascular endothelial cell or the cultured cell derived from the vascular endothelial cell whose expression level of the candidate antiangiogenic gene is altered; and
a signal detection step of detecting phosphorylation of a protein, and
wherein the protein whose phosphorylation is detected in the signal detection step is at least one of a double stranded RNA-dependent protein kinase PKR and a eukaryotic translation initiation factor eIF2α.

10. The method for screening for the antiangiogenic signal gene recited in claim 8 or 9, wherein at least one of phosphorylation of Thr451 of a human double stranded RNA-dependent protein kinase PKR or phosphorylation of a corresponding amino acid residue of a double stranded RNA-dependent protein kinase PKR, and phosphorylation of Ser51 of a human eukaryotic translation initiation factor eIF2α or phosphorylation of a corresponding amino acid residue of a eukaryotic translation initiation factor eIF2α, is detected in the signal detection step.

11. The method for screening for the antiangiogenic signal gene recited in any one of claims 7 to 10, wherein the expression level alteration step is an up-regulating step overexpressing the candidate antiangiogenic signal gene in the vascular endothelial cell or in the cultured cell derived from the vascular endothelial cell, or a down-regulating step repressing expression of the candidate antiangiogenic signal gene in the vascular endothelial cell or in the cultured cell derived from the vascular endothelial cell.

12. The method for screening for the antiangiogenic signal gene recited in any one of claims 7 to 11, wherein the vascular endothelial cell or the cultured cell derived from the vascular endothelial cell is selected from a group comprising capillary vessel endothelial cells, great vessel-umbilical vein endothelial cells and retina vessel endothelial cells.

13. The method for screening for the antiangiogenic signal gene recited in any one of claims 7 to 12, wherein the signal detection step for detecting the phosphorylation of the protein employs at least one of detection methods selected from a group comprising immunoblotting using a phospho-specific antibody, autoradiography using ³²P, immuno-histochemistry using a phospho-specific antibody, gel-shift, and immunoprecipitation using a specific antibody and a phospho-specific antibody.
